(19) Europäisches Patentamt
European Patent Office
Office européen des brevets

(11) Veröffentlichungsnummer : **0 085 763**
**B1**

(12) # EUROPÄISCHE PATENTSCHRIFT

(45) Veröffentlichungstag der Patentschrift : **05.06.85**

(51) Int. Cl.⁴ : **C 07 C175/00**, C 07 F 9/54, C 25 B 3/04

(21) Anmeldenummer : **82111006.1**

(22) Anmeldetag : **29.11.82**

(54) **Verfahren zur Herstellung von Cyclohexenderivaten.**

(30) Priorität : **09.02.82 CH 784/82**

(43) Veröffentlichungstag der Anmeldung : **17.08.83 Patentblatt 83/33**

(45) Bekanntmachung des Hinweises auf die Patenterteilung : **05.06.85 Patentblatt 85/23**

(84) Benannte Vertragsstaaten : **AT BE CH DE FR GB IT LI NL**

(56) Entgegenhaltungen :
**EP-A- 0 031 875**
**EP-A- 0 077 439**
**HELVETICA CHIMICA ACTA, Band 65, Fasc. 3, Nr. 89, 1982, Seiten 958-967, Schweizerische Chemische Gesellschaft, Basel, CH. E. WIDMER et al.: "Technische Verfahren zur Synthese von Carotinoiden und verwandten Verbindungen aus 6-Oxo-isophoron. VII. Synthese von Rhodoxanthin und (3RS,3'RS)-Zeaxanthin aus der C15-Ringkomponente"**

(73) Patentinhaber : **F. HOFFMANN-LA ROCHE & CO. Aktiengesellschaft**
**CH-4002 Basel (CH)**

(72) Erfinder : **Grass, Hansjörg, Dr.**
**Wiesengrundstrasse 7**
**CH-4132 Muttenz (CH)**
Erfinder : **Zell, Reinhard, Dr.**
**Im Zwären**
**CH-4118 Rodersdorf (CH)**

(74) Vertreter : **Cottong, Norbert A. et al**
**Grenzacherstrasse 124 Postfach 3255**
**CH-4002 Basel (CH)**

**EP 0 085 763 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein neues Verfahren zur Herstellung von Cyclohexenderivaten, nämlich von Cyclohexenderivaten, welche als Zwischenprodukte für die Herstellung von Rhodoxanthin bzw. Zeaxanthin geeignet sind, sowie ein Verfahren zur Herstellung von Rhodoxanthin bzw. von Zeaxanthin selbst.

Unter dem Ausdruck Zeaxanthin ist im Rahmen der vorliegenden Erfindung das (3RS, 3'RS)-Zeaxanthin zu verstehen.

Rhodoxanthin ist ein natürlich vorkommendes Carotinoid, welches u. a. als Lebensmittelfarbstoff verwendet werden kann. Auch Zeaxanthin, dessen (3R, 3'R)-Antipode in der Natur vorkommt, kann als Lebensmittelfarbstoff dienen, z. B. zur Eidotterpigmentierung.

Bisher bekannte Synthesen zur Herstellung dieser beiden Carotinoide benötigen etwa 10 bis 18 Stufen. Mittels des erfindungsgemässen Verfahrens können nunmehr diese Substanzen auf wesentlich einfachere Art hergestellt werden.

Das erfindungsgemässe Verfahren ist nun dadurch gekennzeichnet, dass man die Verbindung der Formel

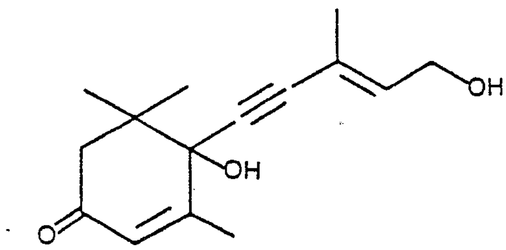

(I)

in einem basischen, wässrig-organischen Lösungsmittelgemisch kathodisch reduziert zur Verbindung der Formel

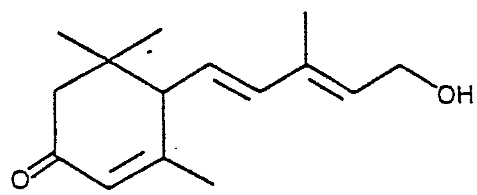

(II)

dass man, gewünschtenfalls, die Verbindung der Formel II, nach Ueberführung in das Phosphoniumsalz der Formel

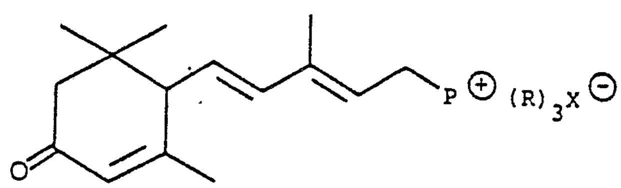

(III)

worin R Phenyl und X Chlor, Brom oder Jod bedeuten, mit dem Dialdehyd der Formel

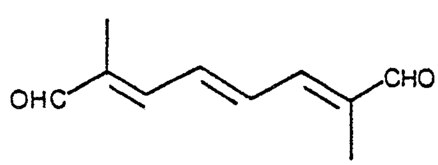

(IV)

zur Verbindung der Formel

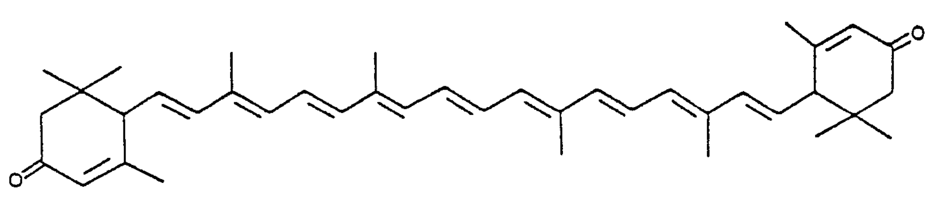

(V)

umsetzt, dass man, gewünschtenfalls, die Verbindung der Formel V zu Rhodoxanthin der Formel

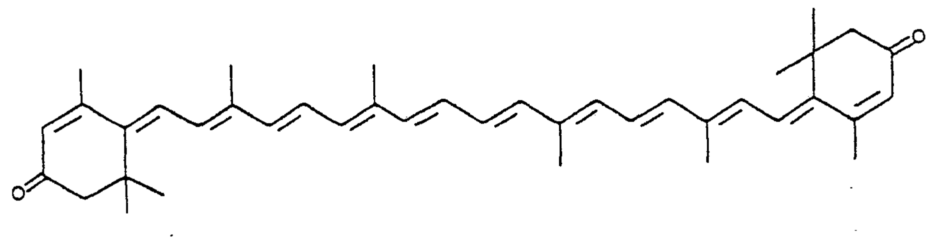

(VI)

dehydriert und dass man, gewünschtenfalls, dieses Rhodoxanthin zu Zeaxanthin der Formel

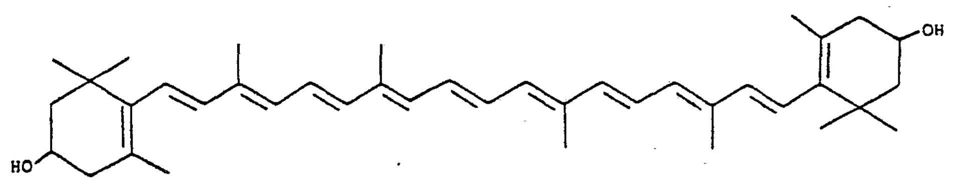

(VII)

reduziert.

Die Ueberführung der Verbindung der Formel I in diejenige der Formel II stellt eine reduktive Eliminierung der angulären Hydroxygruppe und gleichzeitige Partialreduktion der Dreifachbindung dar.

Diese Ueberführung erfolgt erfindungsgemäss durch kathodische Reduktion in einem basischen, wässrig-organischen Lösungsmittelgemisch.

Die erfindungsgemässe Reduktion der Verbindung der Formel I kann in ungeteilten oder unterteilten Zellen durchgeführt werden. Vorzugsweise wird jedoch eine unterteilte Zelle verwendet, wobei die Unterteilung mit Membranen oder Diaphragmen aus üblichen Membran- bzw. Diaphragma-Materialien, beispielsweise Ton, Keramik, Glas (z. B. Glassinterdiaphragma) oder polymeren Verbindungen (z. B. NAFION® [Dupont]) erfolgen kann.

Die Elektroden können übliche Formen aufweisen. Beispielsweise können die Elektroden in Form von Platten oder Gittern oder als Streckmetall ausgebildet sein. Die verwendeten Anoden- und Kathodenmaterialien sind nicht kritisch.

Als Kathodenmaterialien werden vorzugsweise Quecksilber (beispielsweise als Quecksilbersumpfelektrode), Blei, Cadmium, Zinn, Zink, Graphit, Kupfer, Silber, Chromnickelstahl, Messing, Vanadium, Kobalt, Platin, Nickel und dergleichen verwendet. Besonders bevorzugte Kathodenmaterialien sind Blei und Graphit.

Geeignete Anodenmaterialien sind beispielsweise Platin, Graphit, Nickel, Eisen, Kobalt, Blei, Chrom, Chromnickelstahl oder Kupfer. Ferner können auch dimensionsstabile Anoden [wie z. B. die in A. Schmidt, Angewandte Elektrochemie, S. 70, Verlag Chemie (1976) erwähnten], auch Metalloxid-Verbundanoden genannt, verwendet werden. Solche Verbundanoden bestehen aus einem Träger aus Titan, Eisen, Nickel, Kupfer, Graphit, Tantal und dergleichen, welcher mit einer Metalloxidauflage (z. B. Bleidioxid, Rutheniumdioxid oder Mangandioxid) versehen ist, wobei auf der Oberfläche des Trägers vor der Aufbringung des Metalloxidüberzuges eine Zwischenschicht aus einem Carbid oder Borid der Elemente der IV. und V. Nebengruppe aufgebracht wird.

Die kathodische Reduktion der Verbindung der Formel I wird erfindungsgemäss in einem basischen, wässrig-organischen Lösungsmittelgemisch bestehend aus einem wässrigen Elektrolyten (wässrige Komponente) und einem inerten organischen Lösungsmittel (organische Komponente) durchgeführt.

Als wässriger Elektrolyt kann eine wässrige Lösung einer Base, vorzugsweise einer anorganischen Base, wie Natriumhydroxid, Kaliumhydroxid, Lithiumhydroxid, Ammoniak und dergleichen, verwendet werden. Besonders bevorzugt ist Natronlauge. Die Konzentration ist nicht kritisch. Im allgemeinen wird jedoch mit etwa 0,05N bis etwa 10N Lösungen gearbeitet. Vorzugsweise werden etwa 0,1-1N und insbesondere etwa 0,1-0,3N Lösungen verwendet. Bei Verwendung verdünnter Lösungen bzw. bei Verwendung von Ammoniak als Base kann zur Verbesserung der Leitfähigkeit ein Leitsalz zugesetzt werden. Hierzu kommen übliche anorganische Salze, wie Natriumchlorid, Natriumsulfat, Kaliumchlorid, Kaliumbromid, Lithiumchlorid, Ammoniumchlorid und dergleichen, oder auch organische Salze, wie beispielsweise Tetrabutylammoniumhydroxid, Tetraäthylammoniumtosylat oder Tetrabutylammoniumbromid in Betracht.

Das inerte organische Lösungsmittel kann wassermischbar oder nicht wassermischbar sein. Beispiele geeigneter Lösungsmittel sind niedere Alkohole (mit 1-4 Kohlenstoffatomen), cyclische Aether, Aceton, 1,2-Dichloräthan, Tetrachlorkohlenstoff und dergleichen. Bevorzugte organische Lösungsmittel sind die niederen Alkohole, wie Methanol, Aethanol, Isopropanol und t-Butanol, und insbesondere die cyclischen Aether, wie 1,4-Dioxan und Tetrahydrofuran.

Im Falle von 2-phasigen Lösungsmittelgemischen wird dem Reaktionsgemisch zweckmässig ein

Phasentransferkatalysator zugesetzt. Geeignete Phasentransferkatalysatoren sind beispielsweise die Tetraalkylammoniumsalze, wie Tetramethylammoniumtetrafluoroborat, Tetraäthylammoniumtosylat, Tetrabutylammoniumperchlorat, Tetrabutylammoniumbromid, Tetrakis-decyl-ammoniumperchlorat, Hexadecyl-trimethylammoniumbromid und insbesondere die Tetraalkylammoniumhydroxide, wie Tetraäthylammoniumhydroxid, Tetrabutylammoniumhydroxid und dergleichen. Der Anteil solcher Verbindungen richtet sich nach der gewünschten Leitfähigkeit, dem verwendeten Lösungsmittelgemisch und dergleichen.

Das optimale Volumenverhältnis Elektrolyt/organisches Lösungsmittel kann je nach Edukt-Konzentration, verwendetem Lösungsmittelgemisch und dergleichen verschieden sein. Im allgemeinen liegt es jedoch zwischen 6 : 1 und 1 : 5 und vorzugsweise zwischen 6 : 1 und 1 : 1.

Die Konzentration des Ausgangsmaterials der Formel I im verwendeten Reaktionsgemisch ist nicht kritisch. Sie kann im allgemeinen zwischen etwa 0,4 und etwa 6 % (Gew./Vol.) schwanken.

Die bei der Reduktion der Verbindung der Formel I angewandte Temperatur ist nicht kritisch. Sie wird jedoch nach oben hin durch den Siedepunkt des Reaktionsgemisches begrenzt. Vorzugsweise arbeitet man zwischen Raumtemperatur und etwa 70 °C. Die Umsetzung kann mit oder ohne Schutzgasatmosphäre durchgeführt werden. Vorzugsweise arbeitet man unter einer Inertgasatmosphäre, wie etwa Stickstoff, Argon und dergleichen.

Die erfindungsgemässe kathodische Reduktion der Verbindung der Formel I kann galvanostatisch oder potentiostatisch durchfgeführt werden. Das potentiostatische Verfahren ist bevorzugt.

Das erforderliche Potential ist abhängig vom verwendeten Reaktionsgemisch und Kathodenmaterial und kann durch Messung der Strom-Potential-Kurven (z. B. durch cyclische Voltammetrie) ermittelt werden. Im allgemeinen liegt es zwischen etwa − 1 500 und etwa − 1 900 mV (gegen eine gesättigte Kalomelelektrode gemessen).

Die Stromdichte ist nicht kritisch. Zweckmässigerweise wird bei einer Stromdichte von etwa 2-50 $mA/cm^2$ und vorzugsweise etwa 2-10 $mA/cm^2$ gearbeitet.

Die Zellspannung und die Stromstärke sind vom verwendeten Reaktionsgemisch, der Grösse der Zelle, der angewendeten Stromdichte und dergleichen abhängig und können daher stark variieren.

Bei der Ueberführung der Verbindung der Formel II in das Phosphoniumsalz der Formel III wird in der Verbindung der Formel II zunächst die allylische Hydroxylgruppe gegen Halogen ausgetauscht. Dieser Austausch kann in an sich bekannter Weise mittels Halogenwasserstoff (HCl, HBr oder HJ) in wässriger Lösung (z. B. 37 %ig, 48 %ig oder 57 %ig) erfolgen. Der Austausch kann bei Temperaturen zwischen etwa − 20 °C und etwa + 25 °C erfolgen, vorzugsweise bei etwa 0 °C. Als Lösungsmittel für die Durchführung der Reaktion kann ein für derartige Austauschreaktionen geeignetes Lösungsmittel verwendet werden, beispielsweise ein chlorierter Kohlenwasserstoff, wie Methylenchlorid oder Chloroform und dergleichen.

Die Umsetzung des so erhaltenen Halogenids zum Phosphoniumsalz der Formel III kann in an sich bekannter Weise erfolgen. Zweckmässig erfolgt die Umsetzung mit einem Triarylphosphin, insbesondere mit Triphenylphosphin, in einem geeigneten inerten organischen Lösungsmittel, wie etwa einem chlorierten Kohlenwasserstoff, z. B. Methylenchlorid oder Chloroform, oder einem Ester niederer Carbonsäuren mit 1 bis 4 Kohlenstoffatomen, wie Aethylformiat, Aethylacetat usw. Weitherhin erfolgt die Umsetzung vorzugsweise in einer Inertgasatmosphäre und bei etwa Raumtemperatur oder auch bei erhöhter Temperatur. Die Temperatur ist jedoch keine kritische Grösse in dieser Reaktion.

Die Umsetzung des Phosphoniumsalzes der Formel III mit dem Dialdehyd der Formel IV zur Verbindung der Formel V kann in an sich bekannter Weise, d. h. unter den bei Wittig-Reaktionen üblichen Bedingungen durchgeführt werden. Zweckmässig erfolgt die Umsetzung in einem chlorierten Kohlenwasserstoff, wie z. B. Methylenchlorid oder Chloroform und in Gegenwart einer Base, wie z. B. Natriummethylat. Es ist hierbei vorteilhaft, die Base nicht auf einmal, sondern langsam und kontinuierlich zu dem Reaktionsgemisch zu geben.

Die Dehydrierung der Verbindung der Formel V zum Rhodoxanthin der Formel VI kann in an sich bekannter Weise durchgeführt werden, beispielsweise gemäss der von Kuhn und Brockmann in Ber. *66*, 1319 (1933) beschriebenen Oxidation von Dihydrorhodoxanthin zu Rhodoxanthin.

Die Reduktion von Rhodoxanthin der Formel VI zu Zeaxanthin der Formel VII kann ebenfalls in an sich bekannter Weise durchgeführt werden, beispielsweise gemäss Karrer und Solmssen, Helv. Chim. Acta *18*, 477 (1935).

Die in dem erfindungsgemässen Verfahren als Ausgangsmaterial verwendete Verbindung der Formel I kann ausgehend vom Ketoisophoron der Formel

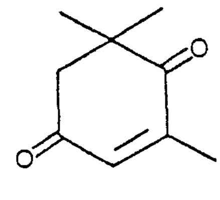

(VIII)

durch Umsetzung mit der Verbindung der Formel

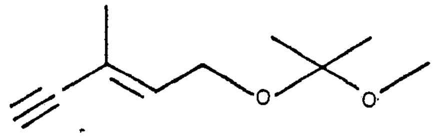

(IX)

hergestellt werden.

Diese Umsetzung erfolgt zweckmässig über das Lithium- oder Magnesiumsalz der Verbindung IX in einem für Organometallverbindungen inerten organischen Lösungsmittel, wie z. B. offene oder cyclische Aether, wie Diäthyläther, Dioxan, Tetrahydrofuran oder auch aromatische Kohlenwasserstoffe, wie Benzol, Toluol oder dergleichen, oder auch in flüssigem Ammoniak. Die Umsetzung erfolgt vorzugsweise bei einer Temperatur von etwa – 50 °C bis etwa Raumtemperatur. Das bei der Umsetzung eingesetzte Ketoisophoron muss in Stellung 3 geschützt sein, z. B. in Form des Lithiumenolates oder als Monoacetal und dergleichen.

Die Erfindung betrifft ferner alle neuen Verbindungen, Mischungen, Verfahren und Verwendungen wie hierin beschrieben.

Das erfindungsgemässe Verfahren wird durch die folgenden Beispiele weiter veranschaulicht.

Beispiel 1

Als Reaktionsgefäss diente ein zweiteiliges Glasgefäss (H-Zelle). Der Anodenraum wurde vom Kathodenraum durch eine runde Polymer-Membran (Durchmesser 7 cm) getrennt. Als Kathode wurde ein Blei-Blech (5,5 cm × 7,5 cm), als Referenzelektrode (im Kathodenraum) eine gesättigte Silber/Silberchlorid-Elektrode (SSE) und als Anode ein Platin-Blech (2,5 cm × 2,5 cm) verwendet. Beide Elektrodenräume wurden je mit einem Gaseinleitungsrohr und der Kathodenraum mit einem Magnetrührer versehen.

Unter Rühren und Stickstoffeinleitung wurden 230 ml 1,4-Dioxan, 230 ml 0,1N Natronlauge sowie 12,4 g 4-Hydroxy-4-(5-hydroxy-3-methyl-3-penten-1-inyl)-3,5,5-trimethyl-2-cyclohexen-1-on in den Kathodenraum und 230 ml 1,4-Dioxan sowie 230 ml 0,1N Natronlauge in den Anodenraum gegeben. Anschliessend wurde bei einem Kathodenpotential von – 1 550 mV (gegen SSE) unter Rühren und Stickstoffeinleitung bei Raumtemperatur elektrolysiert, wobei sich eine Stromstärke von ca. 120 mA einstellte. Der Verlauf der Elektrolyse wurde laufend mittels Dünnschichtchromatographie verfolgt. Nachdem die Stromstärke auf 10 mA abgesunken war und eine Strommenge von 17 400 Coulomb (90 % der Theorie) durch die Zelle geflossen war, wurde die Katholyt-Lösung in einem Scheidetrichter $S_1$ mit 30 ml 3N Schwefelsäure sauer gestellt. Zwei weitere Scheidetrichter $S_2$ und $S_3$ wurden mit je ca. 300 ml halbgesättigter Kochsalzlösung beschickt. Durch die drei Scheidetrichter $S_1$-$S_3$ wurden der Reihe nach drei Portionen zu je 150 ml Methylenchlorid geschickt. Die organischen Phasen wurden über Natriumsulfat getrocknet und filtriert. Die vereinigten Filtrate wurden im Rotationsverdampfer am Wasserstrahlvakuum bis zur Gewichtskonstanz eingeengt, wobei 12,5 g eines gelbbraunen Harzes erhalten wurden, welches durch Säulenchromatographie auf Kieselgel gereinigt wurde. Als Laufmittel wurde zuerst Methylenchlorid und dann ein Methylenchlorid/Diäthyläther-Gemisch (Volumenverhältnis 19 : 1) verwendet. Die Reinheit der einzelnen Fraktionen wurde mittels Dünnschichtchromatographie kontrolliert. Die reinen Fraktionen wurden vereinigt und am Wasserstrahlvakuum bei 60 °C bis zur Gewichtskonstanz eingeengt. Dabei wurden 6,3 g (54 %) 4-(5-Hydroxy-3-methyl-1,3-pentadienyl)-3,5,5-trimethyl-2-cyclohexen-1-on in Form eines gelben Oeles erhalten. Die Struktur dieser Verbindung wurde durch NMR-Spektren eindeutig charakterisiert.

Das als Ausgangsmaterial verwendete 4-Hydroxy-4-(5-hydroxy-3-methyl-3-penten-1-inyl)-3,5,5-trimethyl-2-cyclohexen-1-on kann wie folgt hergestellt werden :

In einem 750 ml-Sulfierkolben, versehen mit Rührer, Thermometer, Ammoniakkühler und einem 100 ml-Tropftrichter mit Druckausgleich und Begasungsaufsatz, wurden 250 ml Ammoniak kondensiert und dann mit 0,1 g Eisen (III) nitrat versetzt. Hierauf wurden in ca. 15 Minuten insgesamt 3,2 g Lithiumdraht (0,46 g-Atome) in Portionen zugesetzt. Das Gemisch wurde bei – 40 °C bis zum vollständigen Umsatz zu $LiNH_2$ weitergerührt (ca. 20 Minuten). Anschliessend wurde unter Kühlen mit einem Aceton/$CO_2$-Bad bei – 40 °C eine Lösung von 77,4 g Aceton-methyl-3-methyl-2-penten-4-inyl-acetal in 20 ml absolutem Aether in ca. 20 Minuten zugetropft, gefolgt von 30,4 g 2,6,6-Trimethyl-2-cyclohexen-1,4-dion in 20 ml absolutem Aether. Das Gemisch wurde bei – 40 °C bis zum vollständigen Umsatz weitergerührt (ca. 2,5 Stunden). Nun wurden 200 ml absoluter Aether zugesetzt und anschliessend Ammoniak mit Hilfe eines Warmwasser-Bades ausgetrieben. Sobald das Gemisch Raumtemperatur erreicht hatte, wurde mit einem Eisbad wieder auf 0 °C abgekühlt und durch tropfenweisen Zusatz von 100 ml entionisiertem Wasser hydrolysiert. Zur weiteren Aufarbeitung wurde das Reaktionsgemisch mit Hilfe von 100 ml Aether in einen 1-Liter-Scheidetrichter $S_1$ gespült. Zwei weitere 500 ml-Scheidetrichter ($S_2$ und $S_3$) wurden mit je 200 ml Aether beschickt. Hierauf wurden der Reihe nach und unter gutem Durchmischen zunächst die wässrige Phase aus $S_1$ sowie drei Portionen zu je 100 ml halbgesättigter Kochsalzlösung durch die drei Scheidetrichter $S_1$ bis $S_3$ geschickt. Die organischen Extrakte wurden vereint und über 50 g Natriumsulfat getrocknet. Hierauf wurde abgenutscht, das Trocknungsmittel auf der Nutsche mit 50 ml Aether nachgewaschen und die Filtrate im Rotationsverdampfer am Wasserstrahlvakuum bei 30 °C eingeengt. Der Rückstand wurde anschliessend im Rotationsverdampfer mit Trockeneiskühler am Feinvakuum (ca. 0,1 mbar) bei 65 °C bis zur Gewichtskonstanz eingeengt. Man erhielt so 62 g (96,8 %) eines Umsetzungsproduktes in Form eines dunklen Oeles.

Diese 62 g Umsetzungsprodukt wurden zur Hydrolyse in 100 ml Aceton aufgenommen und bei 0 °C unter Rühren und Begasen mit Argon tropfenweise in 15 Minuten mit 50 ml 3N Schwefelsäure versetzt. Das schwarze Reaktionsgemisch wurde mit Hilfe von 100 ml Aether in einen 500 ml-Scheidetrichter $S_1$ übergespült und dann mit 200 ml halbgesättigter Kochsalzlösung versetzt und gut geschüttelt. Zwei weitere 500 ml-Scheidetrichter ($S_2$ und $S_3$) wurden mit je 200 ml Aether beschickt. Hierauf wurden der Reihe nach die wässrige Phase aus $S_1$ sowie zwei Portionen zu je 100 ml halbgesättigter Kochsalzlösung unter gutem Durchmischen durch die drei Scheidetrichter $S_1$ bis $S_3$ geschickt. Die organischen Phasen wurden vereint und über 50 g Natriumsulfat getrocknet. Hierauf wurde abgenutscht, das Trocknungsmittel auf der Nutsche mit 50 ml Aether nachgewaschen und die Filtrate im Rotationsverdampfer am Wasserstrahlvakuum bei 30 °C bis zur Gewichtskonstanz eingeengt. Man erhielt so 49,1 g (99,0 %) 4-Hydroxy-4-(5-hydroxy-3-methyl-3-penten-1-inyl)-3,5,5-trimethyl-2-cyclohexen-1-on in Form eines hellbräunlichen Oeles.

## Beispiel 2

Als Reaktionsgefäss wurde eine thermostatisierbare H-Zelle verwendet. Der Anodenraum wurde vom Kathodenraum durch eine runde Polymer-Membran (Durchmesser 4 cm) getrennt. Als Kathode wurde ein Blei-Blech (3,5 cm × 3,5 cm) und als Anode ein Platin-Draht eingesetzt. Das Kathodenpotential wurde gegen eine gesättigte Kalomelreferenzelektrode (SCE) gemessen. Beide Elektrodenräume wurden je mit einem Gaseinleitungsrohr sowie einem Kühler und der Kathodenraum zudem mit einem Magnetrührer versehen.

Unter Rühren und Stickstoffeinleitung wurden 45 ml Aethanol, 45 ml 0,1N Natronlauge sowie 4,97 g 4-Hydroxy-4-(5-hydroxy-3-methyl-3-penten-1-inyl)-3,5,5-trimethyl-2-cyclohexen-1-on (hergestellt gemäss Beispiel 1) in den Kathodenraum und 45 ml Aethanol sowie 45 ml 0,1N Natronlauge in den Anodenraum gegeben. Das Reaktionsgemisch wurde auf eine konstante Temperatur von 65 °C erwärmt und dann bei einem Kathodenpotential von − 1 900 mV (gegen SCE) unter Rühren und Stickstoffbegasung elektrolysiert, wobei sich eine Stromstärke von 160 mA einstellte. Der Verlauf der Elektrolyse wurde laufend mittels Dünnschichtchromatographie verfolgt. Nachdem die Stromstärke auf 80 mA abgesunken war und eine Strommenge von 6 330 Coulomb (80 % der Theorie) durch die Zelle geflossen war, wurde die Elektrolyse abgebrochen, obwohl das Edukt noch nicht vollständig umgesetzt war. Die Katholytlösung wurde anschliessend in einem Scheidetrichter $S_1$ mit 200 ml halbgesättigter Kochsalzlösung verdünnt. Zwei weitere Scheidetrichter $S_2$ und $S_3$ wurden mit je ca. 150 ml halbgesättigter Kochsalzlösung beschickt. Durch die drei Scheidetrichter $S_1$-$S_3$ wurden der Reihe nach drei Portionen zu je 150 ml Methylenchlorid geschickt und dann die organischen Phasen über Natriumsulfat getrocknet und filtriert. Die vereinigten Filtrate wurden im Rotationsverdampfer bei 60 °C unter Wasserstrahlvakuum bis zur Gewichtskonstanz eingeengt. Dabei wurde ein braunes Oel erhalten, welches auf einer Säule mit Kieselgel chromatographisch gereinigt wurde. Als Laufmittel wurde ein Diäthyläther/Hexan-Gemisch verwendet. Die Reinheit der einzelnen Fraktionen wurde mittels Dünnschichtchromatographie kontrolliert. Die reinen Fraktionen wurden vereinigt (die übrigen Fraktionen wurden nicht aufgearbeitet) und am Wasserstrahlvakuum bei 60 °C bis zur Gewichtskonstanz eingeengt. Dabei wurden 1,8 g (39 %) 4-(5-Hydroxy-3-methyl-1,3-pentadienyl)-3,5,5-trimethyl-2-cyclohexen-1-on in Form eines gelben Oeles erhalten. Die Struktur dieser Verbindung wurde durch Mikroanalyse, IR-, MS- und NMR-Spektren eindeutig charakterisiert. Die obigen Verfahrensbedingungen sind nicht optimiert.

## Beispiel 3

Als Reaktionsgefäss wurde eine thermostatisierbare H-Zelle verwendet. Der Anodenraum war vom Kathodenraum durch eine runde Polymer-Membran (Durchmesser 4 cm) getrennt. Als Kathode wurde eine Graphit-Elektrode (5,5 cm × 7,5 cm) und als Anode ein Platindraht eingesetzt. Das Kathodenpotential wurde gegen eine gesättigte Kalomelreferenzelektrode (SCE) gemessen. Beide Elektrodenräume wurden je mit einem Gaseinleitungsrohr sowie einem Kühler und der Kathodenraum zudem mit einem Magnetrührer versehen.

Unter Rühren und Stickstoffeinleitung wurden 250 ml Tetrahydrofuran, 250 ml 0,2N Natronlauge sowie 5,42 g 4-Hydroxy-4-(5-hydroxy-3-methyl-3-penten-1-inyl)-3,5,5-trimethyl-2-cyclohexen-1-on (hergestellt gemäss Beispiel 1) in den Kathodenraum und 250 ml Tetrahydrofuran sowie 250 ml 0,2N Natronlauge in den Anodenraum gegeben. Das Reaktionsgemisch wurde auf eine konstante Temperatur von 55 °C erwärmt und dann bei einem Kathodenpotential von − 1 600 mV (gegen SCE) unter Rühren und Stickstoffbegasung elektrolysiert, wobei sich eine Stromstärke von 250 mA einstellt. Der Verlauf der Elektrolyse wurde laufend mittels Dünnschichtchromatographie verfolgt. Nachdem die Stromstärke auf 120 mA abgesunken war und eine Strommenge von 12'700 Coulomb (151 % der Theorie) durch die Zelle geflossen war, wurde die Elektrolyse abgebrochen. Die Katholytlösung und die Anolytlösung wurden anschliessend vereinigt und in einem Scheidetrichter $S_1$ mit 50 ml 3N Schwefelsäure sauer gestellt. Zwei weitere Scheidetrichter $S_2$ und $S_3$ wurden mit je ca. 1 l verdünnter Kochsalzlösung beschickt. Durch die

drei Scheidetrichter $S_1$-$S_3$ wurden der Reihe nach drei Portionen zu je 300 ml Methylenchlorid geschickt. Die organischen Phasen wurden über Natriumsulfat getrocknet und filtriert. Die vereinigten Filtrate wurden im Rotationsverdampfer am Wasserstrahlvakuum bis zur Gewichtskonstanz eingeengt, wobei 5,7 g eines braunen Harzes erhalten wurde, welches durch Säulenchromatographie auf Kieselgel gereinigt wurde. Als Laufmittel wurden zuerst Methylenchlorid und anschliessend verschiedene Methylenchlorid/Diäthyläther-Gemische (Volumenverhältnisse : 49 : 1, 19 : 1, 9 : 1) verwendet. Die Reinheit der einzelnen Fraktionen wurde mittels Dünnschichtchromatographie kontrolliert. Die reinen Fraktionen wurden vereinigt und am Wasserstrahlvakuum bei 60 °C bis zur Gewichtskonstanz eingeengt. Dabei wurden 2,75 g (54 %) 4-(5-Hydroxy-3-methyl-1,3-pentadienyl)-3,5,5-trimethyl-2-cyclohexen-1-on in Form eines gelben Oeles erhalten. Die Struktur dieser Verbindung wurde durch Mikroanalyse, NMR-, IR- und MS-Spektren eindeutig charakterisiert.

Beispiel 4

In analoger Weise zu der in den Beispielen 1-3 beschriebenen Methode wurden die in Tabelle 1 aufgeführten Versuche A-N durchgeführt. Als Reaktionsgefäss diente eine durch eine Glasmembran in Anoden- und Kathodenraum unterteilte Zelle. Die Versuche F, J, K und L wurden potentiostatisch und die übrigen Versuche galvanostatisch durchgeführt. In den Versuchen F und J betrug das angelegte Potential (gegen eine gesättigte Kalomelelektrode) − 1 700 mV und in den Versuchen K und L − 1 600 mV. Für die Versuche D und G wurde Blei und für die Versuche M und N Platin als Anodenmaterial verwendet ; die übrigen Versuche wurden mit einer Bleidioxid/Titan-Verbundanode durchgeführt. Bei allen Versuchen wurden je gleiche Mengen Katholyt und Anolyt eingesetzt. Bei den Versuchen B-G, M und N wiesen zudem Anolyt und Katholyt die gleiche Zusammensetzung auf. Bei den Versuchen A und H-L wurde jedoch auf die Verwendung eines organischen Lösungsmittels im Anodenraum verzichtet und als Anolyt lediglich die in der Tabelle als Elektrolyt angegebene wässrige Lösung auch als Anolyt verwendet. Ferner wurden bei Versuch K dem Katholyten 2 g Tetrabutylammoniumhydroxid als Phasentransferkatalysator zugesetzt. Die eingesetzte Menge an 4-Hydroxy-4-(5-hydroxy-3-methyl-3-penten-1-inyl)-3,5,5-trimethyl-2-cyclohexen-1-on betrug für die Versuche F und K je 0,3 g, für Versuch M 0,4 g und für die übrigen Versuche je 0,5 g. Alle Versuche wurden unter Stickstoffatmosphäre und bei Raumtemperatur durchgeführt.

Alle Versuche führten zum gewünschten Produkt 4-(5-Hydroxy-3-methyl-1,3-pentadienyl)-3,5,5-trimethyl-2-cyclohexen-1-on. (Nachweis mittels Dünnschichtchromatographie). Die Reaktionsgemische wurden jedoch nicht aufgearbeitet und die Verfahrensbedingungen nicht optimiert.

(Siehe Tabelle 1 Seite 8 f.)

Tabelle 1

| Versuche | Kathode | Katholyt | | Stromdichte $[mA/cm^2]$ |
|---|---|---|---|---|
| | | Elektrolyt | org. Lösungsmittel | |
| A | Pb | 35 ml 1M $NH_3/NH_4Cl$ | 35 ml $CH_3OH$ | 8 |
| B | Pb | 50 ml 0,5N NaOH | 25 ml t-Butanol | 4 |
| C | Pb | 12,5 ml 6N NaOH | 62,5 ml $CH_3OH$ | 5 |
| D | Hg | 60 ml 0,5N NaOH | 15 ml Isopropanol | 4 |
| E | Messing | 60 ml 0,5N NaOH | 15 ml $CH_3OH$ | 4 |
| F | Pt | 35 ml 1N NaOH | 35 ml Dioxan | ca. 3 |
| G | Cd | 35 ml 0,5N NaOH | 35 ml Aceton | 25 |
| H | C | 60 ml 2N NaOH | 10 ml t-Butanol | 50 |
| I | Sn | 35 ml 1N NaOH | 35 ml Tetrahydrofuran | 20 |
| J | Cu | 35 ml 0,1N NaOH | 35 ml Dioxan | ca. 6,4 |
| K | Pb | 45 ml 0,5N NaOH | 25 ml $ClCH_2CH_2Cl$ | ca. 3 |
| L | Ni | 35 ml 0,1N NaOH | 35 ml Dioxan | ca. 8 |
| M | Pb | 35 ml 1N LiOH | 35 ml Dioxan | 7 |
| N | Pb | 35 ml 1N KOH | 35 ml Dioxan | 7 |

Beispiel 5

A) In einem 1,5-Liter-Sulfierkolben, versehen mit Rührer, Thermometer, 500 ml-Tropftrichter mit Druckausgleich und einer Vorrichtung zur Inertbegasung, wurden 46,6 g (0,2 Mol) 4-(5-Hydroxy-3-methyl-1,3-pentadienyl)-3,5,5-trimethyl-2-cyclohexen-1-on (hergestellt gemäss einem der Beispiele 1-3) in 200 ml Methylenchlorid gelöst, vorgelegt und bei − 5 °C bis 0 °C in ca. 20 Minuten unter gutem Rühren und Begasen mit Argon tropfenweise mit insgesamt 124 ml 37 %iger Salzsäure versetzt. Hierauf wurde das Gemisch noch 15 Minuten bei 0 °C bis zum vollständigen Umsatz nachgerührt. Anschliessend wurde das dunkle Reaktionsgemisch mit Hilfe von 200 ml Methylenchlorid in einen 1-Liter-Scheidetrichter $S_1$ übergeführt. Zwei weitere 1-Liter-Scheidetrichter ($S_2$ und $S_3$) wurden mit je 250 ml gesättigter Natriumbicarbonatlösung beschickt. Hierauf wurden die untere organische Phase aus $S_1$, sowie zwei Portionen zu je 250 ml Methylenchlorid unter gutem Durchmischen durch die drei Scheidetrichter $S_1$ bis $S_3$ geschickt. Die organischen Phasen wurden hierauf vereinigt, über 50 g Natriumsulfat getrocknet, abgenutscht und das Trocknungsmittel auf der Nutsche mit 50 ml Methylenchlorid nachgewaschen. Das Filtrat wurde dann im Rotationsverdampfer am Wasserstrahlvakuum bei 30 °C Badtemperatur auf ein Volumen von ca. 100 ml konzentriert.

B) In einem 2,5-Liter-Sulfierkolben, versehen mit Rührer, Thermometer, 250 ml-Tropftrichter mit Druckausgleich und einer Vorrichtung zur Inertbegasung, wurde eine Lösung von 52,4 g (0,2 Mol) Triphenylphosphin in 100 ml Methylenchlorid vorgelegt. Unter Rühren und Inertbegasung wurde dann innert ca. 10 Minuten die gemäss A) erhaltene Methylenchloridlösung zugetropft. Die erhaltene dunkle Lösung wurde anschliessend noch 21 Stunden bei Raumtemperatur gerührt und dann im Rotationsverdampfer am Wasserstrahlvakuum bei 30 °C Badtemperatur auf ein Volumen von ca. 200 ml konzentriert. Anschliessend wurden bei Raumtemperatur unter Rühren und Argonbegasung insgesamt 850 ml Essigester in ca. 30 Minuten zugetropft, wobei bald Auskristallisation begann. Die entstehende Suspension wurde dann 24 Stunden bei Raumtemperatur und 2 Stunden in Eis gerührt, abgenutscht, das Produkt auf der Nutsche mit zwei Portionen zu je 100 ml Essigester gründlich ausgewaschen und dann im Trockenschrank am Wasserstrahlvakuum bei 40 °C bis zur Gewichtskonstanz getrocknet. Die Kristalle wurden anschliessend aus Methylenchlorid/Essigester umkristallisiert, und man erhielt 63,3 g (61,7 %) [3-Methyl-5-(2,6,6-trimethyl-4-oxo-2-cyclohexen-1-yl)-2,4-pentadienyl] triphenylphosphoniumchlorid. Smp. 164-166 °C.

Beispiel 6

A) In einem 50 ml-Mehrhalsrundkolben, versehen mit Rührer, Thermometer, 10 ml-Tropftrichter mit Druckausgleich und einer Vorrichtung zur Inertbegasung, wurden 2,08 g (8,9 mMol) 4-(5-Hydroxy-3-methyl-13-pentadienyl)-3,5,5-trimethyl-2-cyclohexen-1-on (hergestellt gemäss einem der Beispiele 1-3) in 10 ml Methylenchlorid gelöst vorgelegt und bei − 15 °C während 10 Minuten tropfenweise mit insgesamt 4,8 ml 63 %iger Bromwasserstoffsäure versetzt. Danach wurde noch während ca. 30 Minuten bei − 15 °C nachgerührt. Anschliessend wurde das dunkle Reaktionsgemisch mit Hilfe von 25 ml Methylenchlorid in einen 50 ml-Scheidetrichter $S_1$ transferiert. Zwei weitere 50 ml-Scheidetrichter ($S_2$ und $S_3$) wurden mit je 25 ml gesättigter Natriumbicarbonatlösung beschickt. Nun wurden die untere organische Phase aus $S_1$ sowie zwei Portionen zu je 25 ml Methylenchlorid unter gutem Durchmischen durch die drei Scheidetrichter $S_1$ bis $S_3$ geschicht. Die organischen Phasen wurden vereint, über 25 g Natriumsulfat getrocknet, dann abgenutscht, das Trocknungsmittel auf der Nutsche mit 25 ml Methylenchlorid nachgewaschen und das Filtrat im Rotationsverdampfer am Wasserstrahlvakuum bei 30 °C Badtemperatur auf ein Volumen von ca. 10 ml konzentriert.

B) In einem 50 ml-Rundkolben, versehen mit Rührer, Thermometer, 10 ml-Tropftrichter mit Druckausgleich und einer Vorrichtung zur Inertbegasung, wurden 2,89 g (11 mMol) Triphenylphosphin in 10 ml Methylenchlorid vorgelegt. Unter Rühren und Begasen mit Argon wurde dann die Methylenchloridlösung gemäss Abschnitt A) in ca. 10 Minuten zugetropft. Die dunkle Lösung wurde anschliessend während ca. 2 Stunden bei Raumtemperatur nachgerührt und dann im Rotationsverdampfer am Wasserstrahlvakuum bei 30 °C Badtemperatur auf ein Volumen von ca. 20 ml konzentriert. Anschliessend wurden bei Raumtemperatur unter Rühren und Argonbegasung insgesamt 100 ml Essigester in ca. 1 Stunde zugetropft. Hierauf wurde die Suspension angeimpft, 24 Stunden bei Raumtemperatur und 2 Stunden in Eis gerührt und dann abgenutscht. der Rückstand auf der Nutsche wurde mit zwei Portionen zu je 25 ml Essigester gründlich ausgewaschen und dann im Trockenschrank am Wasserstrahlvakuum bei 40 °C bis zur Gewichtskonstanz getrocknet. Man erhielt 4,2 g (84,3 %) [3-Methyl-5-(2,6,6-trimethyl-4-oxo-2-cyclohexen-1-yl)-2,4-pentadienyl] triphenylphosphoniumbromid. Smp. 168-170 °C.

Beispiel 7

A) In einem 1,5-Liter-Sulfierkolben, versehen mit Rührer, Thermometer, 50 ml-Tropftrichter mit Druckausgleich und einer Vorrichtung zur Inertbegasung, wurden 103 g (0,20 Mol) [3-Methyl-5-(2,6,6-trimethyl-4-oxo-2-cyclohexen-1-yl)-2,4-pentadienyl] triphenylphosphonium-chlorid und 13,1 g (79,8 mMol) 2,7-Dimethyl-octatrien-(2,4,6)-dial-(1,8), in 800 ml Methylenchlorid gelöst, vorgelegt. Unter Rühren,

Begasung mit Argon und Kühlen bei – 20 °C wurden nun im Verlauf von 2 Stunden insgesamt 46 ml 10 %ige Natriummethylatlösung zugetropft. Anschliessend wurde noch 2 Stunden bei 0 °C nachgerührt. Die Reaktion wurde durch Zusatz von 12 ml Eisessig gequencht. Drei 2-Liter-Scheidetrichter $S_1$ bis $S_3$ wurden mit je 500 ml gesättigter Kochsalzlösung beschickt. Dann wurden unter kräftigem Durchmischen zunächst die Reaktionslösung und dann zwei Portionen zu je 500 ml Methylenchlorid durch die drei Scheidetrichter $S_1$ bis $S_3$ geschickt. Die organischen Extrakte wurden vereinigt, über 100 g Natriumsulfat getrocknet, abgenutscht und das Trocknungsmittel auf der Nutsche mit zweimal 200 ml Methylenchlorid nachgewaschen.

B) Das gemäss A) erhaltene Filtrat wurde in einen 1,5-Liter-Sulfierkolben, versehen mit Rührer, Thermometer, 250 ml-Tropftrichter mit Druckausgleich und Vorrichtung für Inertbegasung, sowie einem Destillationsaufsatz, gegeben. Unter Rühren und Heizen (120 °C) und unter Argon wurde die Lösung bei Normaldruck auf ein Volumen von ca. 500 ml konzentriert. Dann wurden unter fortgesetzter Destillation bei konstantem Volumen von ca. 500 ml etwa 1,8 l Methanol so lange zugetropft, bis der Siedepunkt von 63 °C erreicht wurde. Während dieses Vorganges kristallisierte ein Produkt aus. Anschliessend wurde der Destillationsaufsatz durch einen Rückflusskühler ersetzt und die Suspension während 3 Tagen am Rückfluss gerührt. Dann wurde das Ganze auf – 20 °C gekühlt und abgenutscht. Die Kristalle wurden auf der Nutsche mit drei Portionen zu je 100 ml Methanol (von – 20 °C) unter Argonbegasung gründlich ausgewaschen, abgepresst und im Trockenschrank unter Wasserstrahlvakuum bei 30 °C bis zur Gewichtskonstanz getrocknet. Man erhielt 32 g (71 %) 1,18-bis(4-oxo-2,6,6-trimethyl-2-cyclohexen-1-yl)-3,7,12,16-tetramethyl-1,3,5,7,9,11,13,15,17-octadecanonaen. Schmelzpunkt 185-187 °C.

In zum Vorhergehenden analoger Weise kann diese Verbindung auch ausgehend von [3-Methyl-5-(2,6,6-trimethyl-4-oxo-2-cyclohexen-1-yl)-2,4-pentadienyl] triphenylphosphonium-bromid hergestellt werden.

Beispiel 8

A) In einem 500 ml-Mehrhalskolben, versehen mit Magnetrührkern, 50 ml-Tropftrichter und Thermometer, wurden 11,3 g (20 mMol) 1,18-bis(4-oxo-2,6,6-trimethyl-2-cyclohexen-1-yl)-3,7,12,16-tetramethyl-1,3,5,7,9,11,13,15,17-octadecanonaen in 200 ml Pyridin bei 70 °C unter Argon gelöst, auf Raumtemperatur gekühlt, unter Luftzutritt 20 Minuten bei 0 °C gerührt, und dann tropfenweise während 10 Minuten mit 50 ml einer 1N äthanolischen Kaliumhydroxidlösung versetzt. Dann wurde noch während 30 Minuten in Eis und während 1,5 Stunden bei Raumtemperatur und freiem Luftzutritt gerührt. Die Reaktionslösung wurde dann mit Hilfe von 200 ml Methylenchlorid in einen 1-Liter-Scheidetrichter $S_1$ transferiert, welcher 400 ml einer 5 %igen Kochsalzlösung enthielt. Zwei weitere 1-Liter-Scheidetrichter ($S_2$ und $S_3$) wurden mit je 500 ml 3N Salzsäure beschickt. Nun wurden die untere, organische Phase aus $S_1$ sowie drei Portionen zu je 200 ml Methylenchlorid unter gutem Durchmischen durch die drei Scheidetrichter ($S_1$ bis $S_3$) geschickt. Die organischen Extrakte wurden mit 400 ml entionisiertem Wasser und 400 ml 2 %iger Natriumbicarbonatlösung gewaschen, über 100 ml Natriumsulfat getrocknet und abgenutscht. Das Trocknungsmittel wurde auf der Nutsche mit zwei Portionen zu je 200 ml Methylenchlorid nachgewaschen und das Filtrat am Rotationsverdampfer am Wasserstrahlvakuum bei 30 °C auf ein Volumen von ca. 100 ml konzentriert.

B) Die gemäss A) erhaltene Methylenchloridlösung (100 ml) wurde in einen 350 ml-Sulfierkolben, versehen mit Rührer, Thermometer, 100 m-Tropftrichter mit Druckausgleich und Vorrichtung für Inertbegasung sowie einem Destillationsaufsatz, gegeben. Unter Rühren und Destillation wurde bei Normaldruck bis auf ein Restvolumen von ca. 100 ml konzentriert. Unter Beibehaltung dieses Volumens wurde anschliessend soviel entionisiertes Wasser zugetropft, bis der Siedepunkt 93 °C (Badtemperatur = 130 °C) erreicht war. Die entstandene klebrige Masse wurde dann 48 Stunden am Rückfluss gerührt, auf Raumtemperatur gekühlt und dann mit Hilfe von ca. 200 ml Methylenchlorid gelöst. Nach dem Trennen der Phasen in einem 500 ml-Scheidetrichter wurde die organische Phase über 25 g Natriumsulfat getrocknet und abgenutscht. Das Trocknungsmittel wurde auf der Nutsche mit zwei Portionen zu je 100 ml Methylenchlorid nachgewaschen und das Filtrat am Rotationsverdampfer am Wasserstrahlvakuum bei 30 °C auf ein Volumen von ca. 50 ml konzentriert. Diese Lösung wurde mit 150 ml Essigester versetzt, worauf das Gemisch im Rotationsverdampfer am Wasserstrahlvakuum bei 40 °C Badtemperatur auf ein Volumen von ca. 50 ml konzentriert wurde. Hierbei begann Produkt auszukristallisieren. Die Suspension wurde anschliessend noch 2 Stunden bei 0 °C gerührt und dann genutscht. Die Kristalle auf der Nutsche wurden mit zwei Portionen zu je 50 ml Essigester gründlich ausgewaschen und dann im Trockenschrank am Wasserstrahlvakuum bei 30 °C bis zur Gewichtskonstanz getrocknet. Man erhielt 6,3 g (56 %) Rhodoxanthin mit einem Schmelzpunkt von 208-210 °C.

**Patentansprüche**

1. Verfahren zur Herstellung von Cyclohexenderivaten, dadurch gekennzeichnet, dass man die

Verbindung der Formel

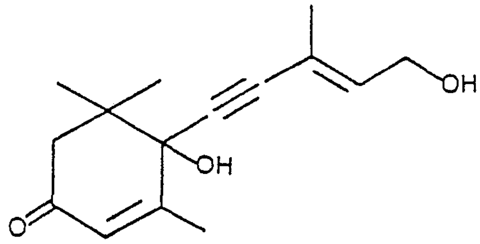

(I)

in einem basischen, wässrig-organischen Lösungsmittelgemisch kathodisch reduziert zur Verbindung der Formel

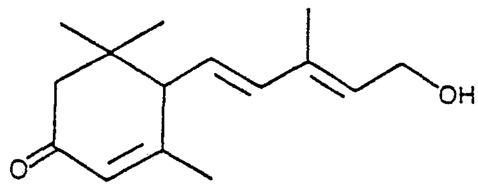

(II)

dass man, gewünschtenfalls, die Verbindung der Formel II, nach Ueberführung in das Phosphoniumsalz der Formel

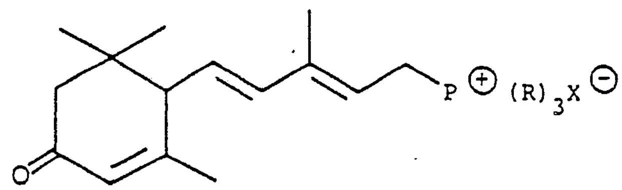

(III)

worin R Phenyl und X Chlor, Brom oder Jod bedeuten, mit dem Dialdehyd der Formel

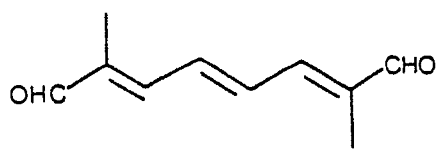

(IV)

zur Verbindung der Formel

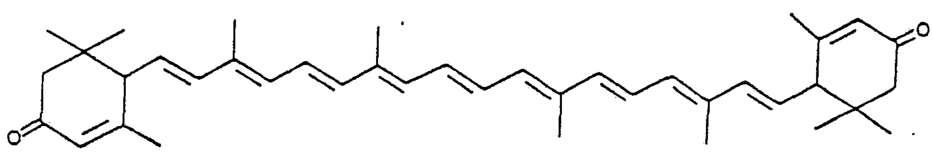

(V)

umsetzt, dass man, gewünschtenfalls, die Verbindung der Formel V zu Rhodoxanthin der Formel

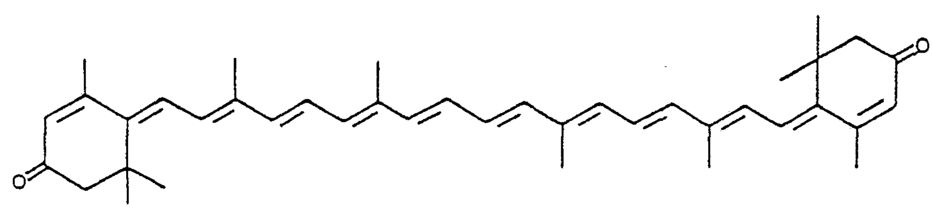

(VI)

dehydriert und dass man, gewünschtenfalls, dieses Rhodoxanthin zu Zeaxanthin der Formel

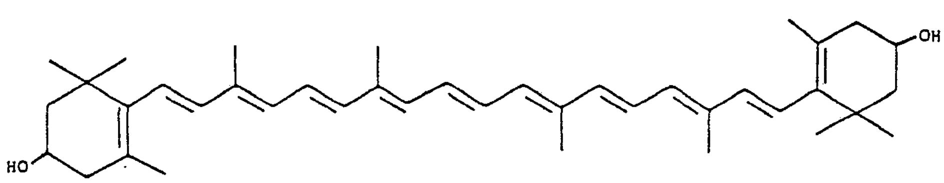

(VII)

reduziert.

2. Verfahren nach Anspruch 1, dadurch gekennzeichnet, dass man die kathodische Reduktion der Verbindung der Formel I in einer unterteilten Zelle durchführt.

3. Verfahren nach Anspruch 1 oder 2, dadurch gekennzeichnet, dass man als wässrige Komponente des wässrig-organischen Lösungsmittelgemisches eine wässrige Lösung einer anorganischen Base, vorzugsweise Natriumhydroxid, Kaliumhydroxid, Lithiumhydroxid oder Ammoniak, verwendet.

4. Verfahren nach Anspruch 3, dadurch gekennzeichnet, dass man als wässrige Komponente des wässrig-organischen Lösungsmittelgemisches Natronlauge verwendet.

5. Verfahren nach Anspruch 3 oder 4, dadurch gekennzeichnet, dass man als wässrige Komponente des wässrig-organischen Lösungsmittelgemisches eine 0,05-10N, vorzugsweise 0,1-1N Lösung der Base verwendet.

6. Verfahren nach einem der Ansprüche 1 bis 5, dadurch gekennzeichnet, dass man als organische Komponente des wässrig-organischen Lösungsmittelgemisches einen Alkohol mit 1 bis 4 Kohlenstoffatomen oder einen cyclischen Aether, vorzugsweise Methanol, Aethanol, Isopropanol, t-Butanol, 1,4-Dioxan oder Tetrahydrofuran verwendet.

7. Verfahren nach Anspruch 6, dadurch gekennzeichnet, dass man als organische Komponente des wässrig-organischen Lösungsmittelgemisches einen cyclischen Aether, vorzugsweise 1,4-Dioxan oder Tetrahydrofuran verwendet.

8. Verfahren nach einem der Ansprüche 1 bis 7, dadurch gekennzeichnet, dass man die kathodische Reduktion in einem wässrig-organischen Lösungsmittelgemisch durchführt, dessen Volumenverhältnis wässrige Komponente/organische Komponente 6 : 1 bis 1 : 5 beträgt.

9. Verfahren nach einem der Ansprüche 1 bis 8, dadurch gekennzeichnet, dass man die kathodische Reduktion bei einer Temperatur zwischen Raumtemperatur und der Siedetemperatur des Reaktionsgemisches durchführt.

10. Verfahren nach einem der Ansprüche 1 bis 9, dadurch gekennzeichnet, dass man als Kathodenmaterial Blei oder Graphit verwendet.

**Claims**

1. A process for the manufacture of cyclohexene derivatives, characterized by cathodically reducing the compound of the formula

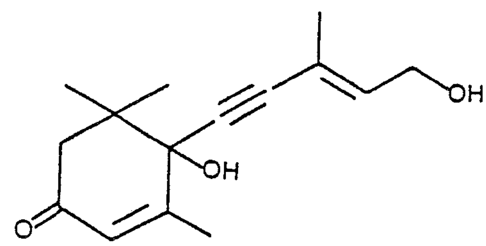

(I)

in a basic, aqueous-organic solvent mixture to give the compound of the formula

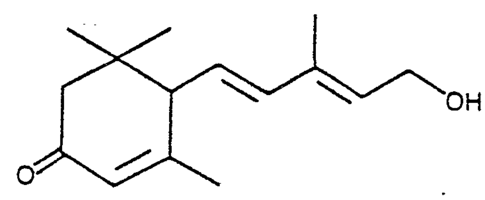

(II)

if desired, reacting the compound of formula II, after conversion into the phosphonium salt of the formula

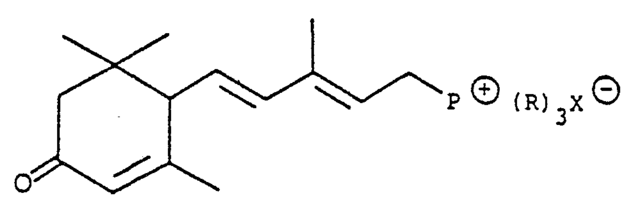

(III)

wherein R signifies phenyl and X signifies chlorine, bromine or iodine, with the dialdehyde of the formula

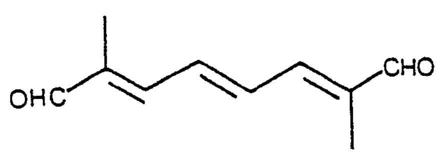

(IV)

to give the compound of the formula

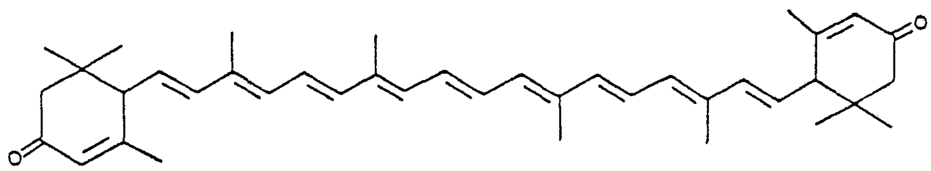

(V)

if desired, dehydrogenating the compound of formula V to give rhodoxanthin of the formula

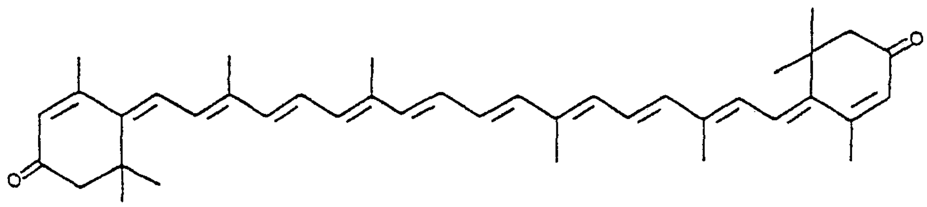

(VI)

and, if desired, reducing this rhodoxanthin to give zeaxanthin of the formula

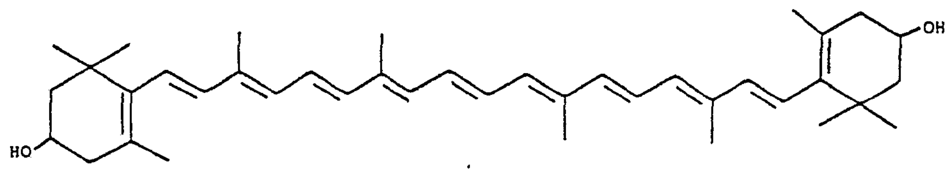

(VII)

2. A process according to claim 1, characterized in that the cathodic reduction of the compound of formula I is carried out in a partitioned cell.

3. A process according to claim 1 or 2, characterized in that an aqueous solution of an inorganic base, preferably sodium hydroxide, potassium hydroxide, lithium hydroxide or ammonia, is used as the aqueous component of the aqueous-organic solvent mixture.

4. A process according to claim 3, characterized in that sodium hydroxide solution is used as the aqueous component of the aqueous-organic solvent mixture.

5. A process according to claim 3 or 4, characterized in that a 0.05-1.0N, preferably 0.1-1N, solution of the base is used as the aqueous component of the aqueous-organic solvent mixture.

6. A process according to any one of claims 1 to 5, characterized in that an alcohol with 1 to 4 carbon atoms or a cyclic ether, preferably methanol, ethanol, isopropanol, t-butanol, 1,4-dioxan or tetrahydrofuran, is used as the organic component of the aqueous-organic solvent mixture.

7. A process according to claim 6, characterized in that a cyclic ether, preferably 1,4-dioxan or tetrahydrofuran, is used as the organic component of the aqueous-organic solvent mixture.

8. A process according to any one of claims 1 to 7, characterized in that the cathodic reduction is carried out in an aqueous-organic solvent mixture whose volume ratio aqueous component/organic component amounts to 6 : 1 to 1 : 5.

9. A process according to any one of claims 1 to 8, characterized in that the cathodic reduction is carried out at a temperature between room temperature and the boiling temperature of the reaction mixture.

10. A process according to any one of claims 1 to 9, characterized in that lead or graphite is used as the cathode material.

**Revendications**

1. Procédé de préparation de dérivés du cyclohexène, caractérisé en ce que l'on soumet le composé de formule

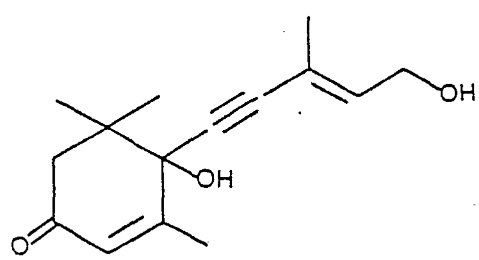

(I)

à réduction cathodique dans un mélange solvant hydroorganique basique, ce qui donne le composé de formule

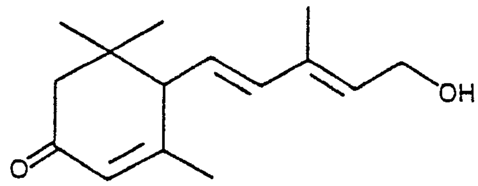

(II)

qu'on fait réagir si on le désire, après conversion en le sel de phosphonium de formule

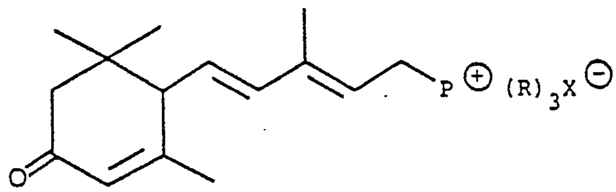

(III)

dans laquelle R représente un groupe phényle et X le chlore, le brome ou l'iode, avec le dialdéhyde de formule

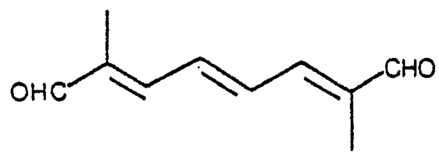

(IV)

ce qui donne le composé de formule

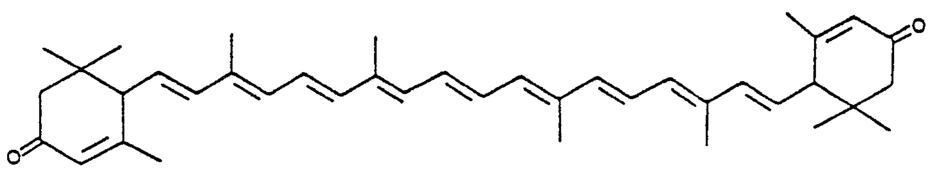

(V)

qu'on convertit si on le désire, par déshydrogénation, en la rhodoxanthine de formule

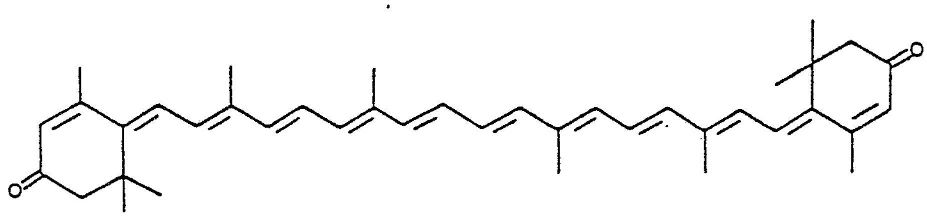

(VI)

qu'on réduit si on le désire en la zéaxanthine de formule

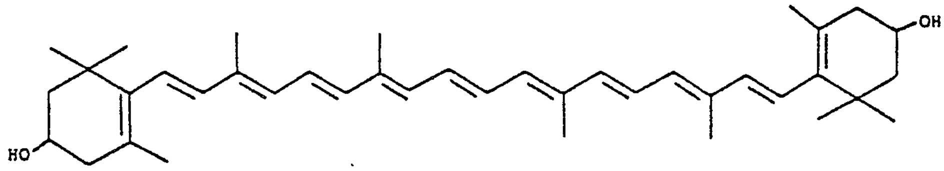

(VII)

2. Procédé selon la revendication 1, caractérisé en ce que la réduction cathodique du composé de formule I est effectuée dans une cellule compartimentée.

3. Procédé selon la revendication 1 ou 2, caractérisé en ce que l'on utilise en tant que composé aqueux du mélange solvant hydroorganique une solution aqueuse d'une base minérale, de préférence l'hydroxyde de sodium, l'hydroxyde de potassium, l'hydroxyde de lithium ou l'ammoniac.

4. Procédé selon la revendication 3, caractérisé en ce que l'on utilise en tant que composant aqueux du mélange solvant hydroorganique de la lessive de soude.

5. Procédé selon la revendication 3 ou 4, caractérisé en ce que l'on utilise en tant que composant aqueux du mélange solvant hydroorganique une solution 0,05 à 10N, de préférence 0,1 à 1N, de la base.

6. Procédé selon l'une des revendications 1 à 5, caractérisé en ce que l'on utilise en tant que composant organique du mélange solvant hydroorganique un alcool en $C_1$-$C_4$ ou un éther cyclique, de préférence le méthanol, l'éthanol, l'isopropanol, le tert-butanol, le 1,4-dioxanne ou le tétrahydrofuranne.

7. Procédé selon la revendication 6, caractérisé en ce que l'on utilise en tant que composant organique du mélange hydroorganique un éther cyclique, de préférence le 1,4-dioxanne ou le tétrahydrofuranne.

8. Procédé selon l'une des revendications 1 à 7, caractérisé en ce que l'on procède à la réduction cathodique dans un mélange solvant hydroorganique dont les proportions en volume composant aqueux/composant organique sont de 6 : 1 à 1 : 5.

9. Procédé selon l'une des revendications 1 à 8, caractérisé en ce que l'on procède à la réduction cathodique à une température comprise entre la température ambiante et la température d'ébullition du mélange de réaction.

10. Procédé selon l'une des revendications 1 à 9, caractérisé en ce que l'on utilise en tant que matériau de cathode le plomb ou le graphite.